(19) Europäisches Patentamt
European Patent Office
Office européen des brevets

(11) **EP 3 854 397 A1**

(12) **EUROPEAN PATENT APPLICATION**
published in accordance with Art. 153(4) EPC

(43) Date of publication:
**28.07.2021 Bulletin 2021/30**

(21) Application number: **19863743.1**

(22) Date of filing: **17.09.2019**

(51) Int Cl.:
***A61K 31/4709** (2006.01)*
***A61K 31/436** (2006.01)*
***A61K 31/44** (2006.01)*
***A61K 31/4545** (2006.01)*
***A61K 31/47** (2006.01)*
***A61K 31/506** (2006.01)*
***A61K 31/517** (2006.01)*
***A61K 31/519** (2006.01)*
***A61K 31/5377** (2006.01)*
***A61K 45/00** (2006.01)*
***A61P 1/02** (2006.01)*
***A61P 1/16** (2006.01)*
***A61P 11/00** (2006.01)*
***A61P 15/00** (2006.01)*
***A61P 17/00** (2006.01)*
***A61P 35/00** (2006.01)*
***A61P 35/02** (2006.01)*
***A61P 43/00** (2006.01)*

(86) International application number:
**PCT/JP2019/036363**

(87) International publication number:
**WO 2020/059705 (26.03.2020 Gazette 2020/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.09.2018 JP 2018174179**

(71) Applicants:
- **Kabushiki Kaisha Yakult Honsha**
  **Tokyo, 105-8660 (JP)**
- **General Incorporated Association Pharma Valley Project Supporting Organization**
  **Mishima-shi, Shizuoka 411-0033 (JP)**

(72) Inventors:
- **ASAI, Akira**
  **Shizuoka-shi, Shizuoka 422-8526 (JP)**
- **TSUGANE, Momomi**
  **Tokyo 105-8660 (JP)**
- **KONISHI, Hiroaki**
  **Tokyo 105-8660 (JP)**
- **YOSHINAGA, Akiko**
  **Tokyo 105-8660 (JP)**
- **TAKAHASHI, Hiroyuki**
  **Tokyo 105-8660 (JP)**
- **IIJIMA, Takahiro**
  **Tokyo 105-8660 (JP)**

(74) Representative: **Schiener, Jens**
**Wächtershäuser & Hartz**
**Patentanwaltspartnerschaft mbB**
**Weinstraße 8**
**80333 München (DE)**

# (54) CANCER COMBINATION THERAPY USING QUINOLINE CARBOXAMIDE DERIVATIVE

(57) Provided is a method for using a STAT3 inhibitor having a high antitumor effect and little side effects. The antitumor agent comprises a combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

EP 3 854 397 A1

[Figure 1]

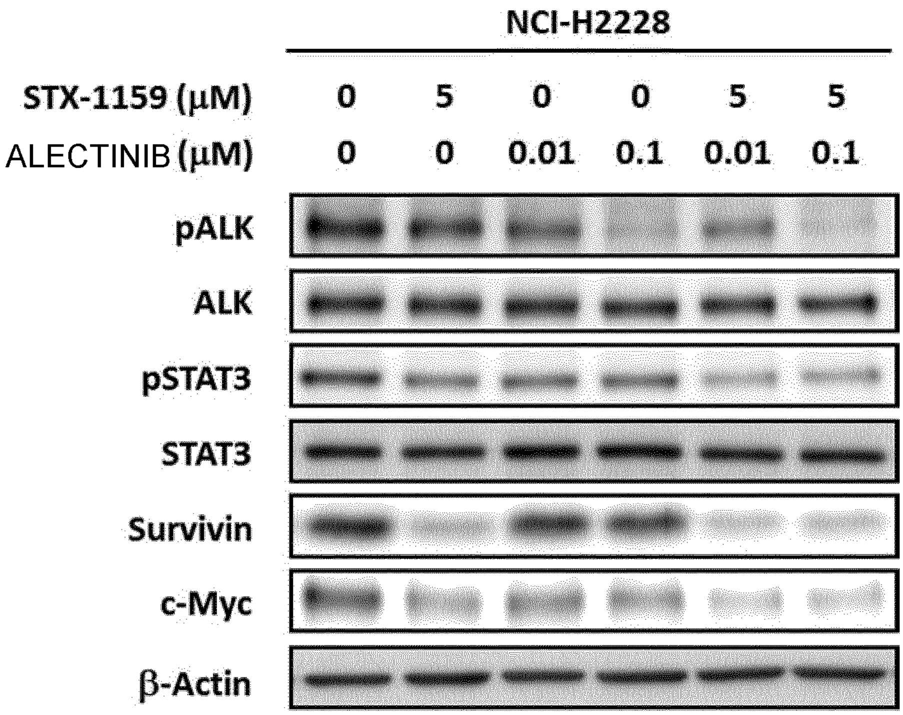
NCI-H2228
STX-1159 (μM) 0 5 0 0 5 5
ALECTINIB (μM) 0 0 0.01 0.1 0.01 0.1
pALK
ALK
pSTAT3
STAT3
Survivin
c-Myc
β-Actin

## Description

### Technical Field

The present invention relates to a cancer combination therapy using a quinoline carboxamide derivative.

### Background Art

STAT (signal transducers and activators of transcription), which is a transcription regulating factor, is a DNA-binding protein, and its activity is regulated by stimuli of a diversity of cytokines (e.g. IL-6 and interferons) or growth factors (e.g. EGF and PDGF). STAT activated by forming a dimer transfers into the nucleus, specifically recognizes a specific DNA sequence in a gene promotor region, and binds to the DNA sequence to induce transcription of many genes. That is, STAT is an essential mediator in a pathway for signaling from the cell surface to the nucleus, and is deeply involved in cell growth and differentiation.
It is known that STAT is classified into seven different members. Of these, STAT3 is expressed in most cell species, and constant activation and excessive expression of STAT3 are observed in cells of cancers such as lung cancer, skin cancer, pancreas cancer, ovary cancer, myeloma, breast cancer, prostate cancer, brain cancer, head and neck cancer, melanoma, leukemia lymphoma and multiple myeloma, and growth and infiltration of these cancer cells are considered to depend on STAT3.
Therefore, STAT3 may be useful as a target molecule for these cancers, and inhibitors of STAT3 are expected as anticancer agents. For example, specific quinoline carboxamide derivatives have been reported to have an excellent STAT3 inhibitory activity, and have antitumor activity against various cancers (Patent Literature 1).

### Citation List

### Patent Literature

Patent Literature 1: JP-B-5650529

## Disclosure of Invention

### Technical Problem

The present invention relates to providing a method for using a STAT3 inhibitor having a high antitumor effect and little side effects.

### Solution to Problem

The present inventors extensively conducted studies in order to further enhance the antitumor effect of a quinoline carboxamide derivative of formula (I) below, and resultantly found that an excellent antitumor effect is obtained by using a specific cancer molecular target drug in combination with the quinoline carboxamide derivative.
That is, the present invention relates to the following 1) to 24).

1) An antitumor agent comprising a combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.
2) The antitumor agent according to 1), which is a kit comprising: an agent containing a quinoline carboxamide derivative of formula (I) or a pharmacologically acceptable salt thereof; and an agent containing one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.
3) The antitumor agent according to 1), which is a combination preparation.
4) The antitumor agent according to any one of 1) to 3), wherein in formula (I), $R^1$ and $R^2$ are the same or different, and each represent a substituted or unsubstituted aryl group or a substituted or unsubstituted aromatic heterocyclic group.
5) The antitumor agent according to 4), wherein in formula (I), at least one of $R^3$, $R^4$, $R^5$ and $R^6$ is a group other than a hydrogen atom.
6) The antitumor agent according to any one of 1) to 5), wherein in $R^1$ and $R^2$ in formula (I), the aryl group is a

phenyl group, and the aromatic heterocyclic group is a furyl group.

7) The antitumor agent according to any one of 1) to 5), wherein in formula (I), $R^1$ is a furyl group, and $R^2$ is a substituted or unsubstituted phenyl group.

8) The antitumor agent according to any one of 1) to 7), wherein at least one of $R^3$, $R^4$, $R^5$ and $R^6$ is a trifluoromethoxy group.

9) The antitumor agent according to any one of 1) to 7), wherein $R^4$ is a trifluoromethoxy group.

10) The antitumor agent according to any one of 1) to 9), wherein the quinoline carboxamide derivative of formula (I) is N-[5-(2-furyl)-1,3,4-oxadiazol-2-yl]-2-phenyl-6-trifluoromethoxy-4-quinoline carboxamide.

11) The antitumor agent according to any one of 1) to 10), wherein the cancer molecular target drug comprises one or more selected from the group consisting of crizotinib, alectinib, ceritinib, osimertinib, sorafenib, vandetanib, lenvatinib, lapatinib, everolimus, dabrafenib, trametinib, imatinib and dasatinib.

12) The antitumor agent according to any one of 1) to 10), wherein the cancer molecular target drug comprises one or more selected from the group consisting of crizotinib, alectinib, ceritinib, osimertinib, sorafenib, vandetanib, lenvatinib, everolimus, dabrafenib, trametinib, imatinib and dasatinib.

13) The antitumor agent according to any one of 1) to 12), wherein the cancer comprises one or more selected from the group consisting of non-small cell lung cancer, tongue cancer, thyroid gland cancer, hepatocyte cancer, breast cancer, ovary cancer, uterine body cancer, melanoma and leukemia.

14) An antitumor agent comprising a quinoline carboxamide derivative of formula (I) below or a pharmacologically acceptable salt thereof as an active ingredient, the antitumor agent being administered in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

15) An antitumor effect enhancer for one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, the antitumor effect enhancer comprising a quinoline carboxamide derivative of formula (I) below or a pharmacologically acceptable salt thereof as an active ingredient.

16) A pharmaceutical composition comprising: a quinoline carboxamide derivative or a pharmacologically acceptable salt thereof; and one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

17) A combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, for use in treating a tumor.

18) A quinoline carboxamide derivative of formula (I) below or a pharmacologically acceptable salt thereof, for use in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, in treating a tumor.

19) A method for treating a tumor, comprising administering to a patient, a therapeutically effective amount of a quinoline carboxamide derivative of formula (I) below or a pharmacologically acceptable salt thereof and one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

20) Use of a combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, for manufacturing an antitumor agent.

21) Use of a quinoline carboxamide derivative of formula (I) below or a pharmacologically acceptable salt thereof, for manufacturing an antitumor agent which is administered in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

22) A quinoline carboxamide derivative of formula (I) below or a pharmacologically acceptable salt thereof, for enhancing an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

23) A method for enhancing an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, the method comprising administering a therapeutically effective amount of a quinoline carboxamide derivative of formula (I) below or a pharmacologically acceptable salt thereof to a patient.

24) Use of a quinoline carboxamide derivative of formula (I) below or a pharmacologically acceptable salt thereof, for manufacturing an antitumor effect enhancer which enhances an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

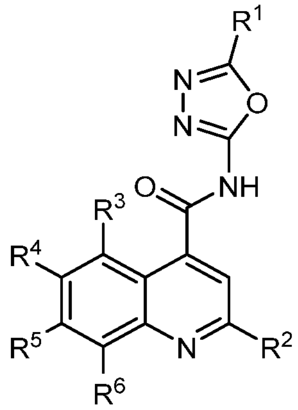

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

Advantageous Effect of Invention

The antitumor agent according to the present invention enables cancer treatment which exhibits a high antitumor effect while suppressing development of side-effects, and therefore a patient can live for a long term.

Brief Description of Drawing

[Figure 1] Figure 1 shows suppression of a STAT3 pathway and an ALK pathway by use of STX-1159 in combination with alectinib.

Description of Embodiments

In a quinoline carboxamide derivative of formula (I), $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

Here, examples of the substituent in the alkyl group include a halogen atom, and a hydroxy group. The substituent in the aryl group or the aromatic heterocyclic group is appropriately selected from the group consisting of an alkyl group having 1 to 6 carbon atoms, an alkenyl group, an alkynyl group, a cycloalkyl group, an aralkyl group, $OR^a$, $NR^bR^c$, $S(O)qR^d$ (wherein q is 0, 1 or 2), $COR^e$, $COOR^f$, $OCOR^g$, $CONR^hR^i$, $NR^jCOR^k$, $NR^lCOOR^m$, $NR^nSO_2R^o$, $C(=NR^p)NR^qR^r$, $NR^sSO_2NR^tR^u$, $SO_2NR^vR^w$, a nitro group, a cyano group, a halogen atom and the like. Here, $R^a$ to $R^w$ may be the same or different, and each represent a hydrogen atom, an alkyl group, an alkenyl group, an alkynyl group, a cycloalkyl group or the like.

The number of substitutions with these substituents, which are the same or different, can be at most equal to the number of hydrogen atoms present in each group, and is preferably from 1 to 10, more preferably from 1 to 5.

Details of the groups specified in formula (I) above are described below. For groups having positional isomers in the groups, all possible positional isomers are shown.

Examples of the alkyl moiety of the alkyl group and the alkoxy group include linear or branched alkyl having 1 to 12 carbon atoms, specifically methyl, ethyl, propyl, isopropyl, butyl, isobutyl, sec-butyl, tert-butyl, pentyl, isopentyl, neopentyl, hexyl, heptyl, octyl, nonyl, decyl, undecyl and dodecyl.

Examples of the cycloalkyl group include three- to twelve-membered cycloalkyl groups which are saturated or optionally have partially unsaturated bonds. The cycloalkyl group may be a monocyclic cycloalkyl group, or a polycyclic

condensed cycloalkyl group in which a plurality of the monocyclic cycloalkyl groups are condensed together, or the monocyclic cycloalkyl group is condensed with an aryl group or an aromatic heterocyclic group. Examples of the monocyclic cycloalkyl group include monocyclic cycloalkyl having 3 to 8 carbon atoms, specifically cyclopropyl, cyclobutyl, cyclopentyl, cyclohexyl, cycloheptyl, cyclooctyl, cyclododecyl and 1-cyclohexenyl. Examples of the polycyclic cycloalkyl group include polycyclic cycloalkyl having 5 to 12 carbon atoms, specifically pinanyl, adamantyl, bicyclo[3.3.1]octyl and bicyclo[3.1.1]heptyl.

Examples of the alkenyl group include linear or branched alkenyl having 2 to 12 carbon atoms, specifically vinyl, allyl, 1-propenyl, isopropenyl, methacryl, butenyl, 1,3-butadienyl, crotyl, pentenyl, hexenyl, heptenyl, decenyl and dodecenyl.

Examples of the alkynyl group include linear or branched alkynyl having 2 to 12 carbon atoms, specifically ethynyl, propargyl, 1-propynyl, isopropynyl, 2-butynyl, pentynyl, 2-penten-4-ynyl, hexynyl, heptynyl, decynyl and dodecynyl.

Examples of the aryl group include aryl having 6 to 14 carbon atoms, specifically phenyl, naphthyl, anthryl and phenanthryl.

Example of the aromatic heterocyclic group includes a five- or six-membered aromatic heterocyclic group containing at least one hetero atoms, for example nitrogen, oxygen or sulfur which are the same or different and the heterocyclic group may be a monocyclic heterocyclic group, or a polycyclic condensed aromatic heterocyclic group in which a plurality of the monocyclic heterocyclic groups are condensed together, or the monocyclic heterocyclic group is condensed with an aryl group, for example a dicyclic or tricyclic heterocyclic group. Specific examples of the monocyclic aromatic heterocyclic group include furyl, thienyl, pyrrolyl, imidazolyl, pyrazolyl, triazolyl, tetrazolyl, oxazolyl, isoxazolyl, oxadiazolyl, thiazolyl, thiadiazolyl, isothiazolyl, pyridyl, pyrimidinyl, pyrazinyl, pyridazinyl and triazinyl. Examples of the polycyclic condensed aromatic heterocyclic group include benzofuryl, benzothienyl, indolyl, isoindolyl, indazolyl, benzoimidazolyl, benzotriazolyl, benzooxazolyl, benzothiazolyl, carbazolyl, purinyl, quinolyl, isoquinolyl, quinazolinyl, phthalazinyl, quinoxalinyl, cinnolinyl, naphthyridinyl, pyridopyrimidinyl, pyrimidopyrimidinyl, pteridinyl, acridinyl, thianthrenyl, phenoxathinyl, phenoxazinyl, phenothiazinyl and phenazinyl.

Examples of halogen atoms include atoms of fluorine, chlorine, bromine and iodine.

The quinoline carboxamide derivative of formula (I) is preferably a compound in which $R^1$ and $R^2$ are the same or different, and each represent a substituted or unsubstituted aryl group, or a substituted or unsubstituted aromatic heterocyclic group. Specifically, preferable specific examples of the aryl group include a phenyl group and a naphthyl group, and preferable specific examples of the aromatic heterocyclic group include a furyl group and a thienyl group. The quinoline carboxamide derivative is more preferably a compound in which $R^1$ represents a furyl group, and $R^2$ represents a substituted or unsubstituted phenyl group, a substituted or unsubstituted furyl group or a substituted or unsubstituted thienyl group. Preferable examples of the substituent in the substituted phenyl group include an alkyl group such as a methyl group, a substituted or unsubstituted alkoxy group such as a methoxy group or a difluoromethoxy group, a halogen atom such as a fluorine atom or a chlorine atom, a hydroxy group, an alkoxycarbonyl group such as a tert-butoxycarbonyl group, an amino group, a nitro group, and a cyano group, and preferable examples of the substituent in the substituted furyl group and the substituted thienyl group include an alkyl group such as a methyl group and a halogen atom such as a chlorine atom.

The quinoline carboxamide derivative is still more preferably a compound in which $R^3$, $R^5$ and $R^6$ each represent a hydrogen atom, and $R^4$ represents $OR^8$ (preferably a methoxy group or a trifluoromethoxy group).

Specific examples of preferred compounds (I) include N-[5-(2-furyl)-1, 3, 4-oxadiazol-2-yl]-2-phenyl-4-quinoline carboxamide, N-[5-(3-furyl)-1,3,4-oxadiazol-2-yl]-2-phenyl-4-quinoline carboxamide, 2-phenyl-N-(5-phenyl-1,3,4-oxadiazol-2-yl)-4-quinoline carboxamide, N-[5-(4-chlorophenyl)-1,3,4-oxadiazol-2-yl]-2-phenyl-4-quinoline carboxamide, N-[5-(4-nitrophenyl)-1,3,4-oxadiazol-2-yl]-2-phenyl-4-quinoline carboxamide, 2-phenyl-N-[5-(3-pyridyl)-1,3,4-oxadiazol-2-yl]-4-quinoline carboxamide, N-[5-(2-furyl)-1,3,4-oxadiazol-2-yl]-2-(3-nitrophenyl)-4-quinoline carboxamide, 2-(4-cyanophenyl)-N-[5-(2-furyl)-1,3,4-oxadiazol-2-yl]-4-quinoline carboxamide, 2-(2-furyl)-N-[5-(2-furyl)-1,3,4-oxadiazol-2-yl]-4-quinoline carboxamide, 2-(5-chloro-2-thienyl)-N-[5-(2-furyl)-1,3,4-oxadiazol-2-yl]-4-quinoline carboxamide, N-[5-(2-furyl)-1,3,4-oxadiazol-2-yl]-6-methoxy-2-phenyl-4-quinoline carboxamide, 2-(1-butoxy)-N-[5-(2-furyl)-1,3,4-oxadiazol-2-yl]-4-quinoline carboxamide, 2-(2-chlorophenyl)-N-[5-(2-furyl)-1,3,4-oxadiazol-2-yl]-4-quinoline carboxamide, N-[5-(2-furyl)-1,3,4-oxadiazol-2-yl]-2-(2-hydroxyphenyl)-4-quinoline carboxamide, 2-(2-aminophenyl)-N-[5-(2-furyl)-1,3,4-oxadiazol-2-yl]-4-quinoline carboxamide, 2-(3-chlorophenyl)-N-[5-(2-furyl)-1,3,4-oxadiazol-2-yl]-4-quinoline carboxamide, N-[5-(2-furyl)-1,3,4-oxadiazol-2-yl]-2-(3-methoxyphenyl)-4-quinoline carboxamide, 2-(3-cyanophenyl)-N-[5-(2-furyl)-1,3,4-oxadiazol-2-yl]-4-quinoline carboxamide, 2-(3-tert-butoxycarbonylphenyl)-N-[5-(2-furyl)-1,3,4-oxadiazol-2-yl]-4-quinoline carboxamide, 2-(4-fluorophenyl)-N-[5-(2-furyl)-1,3,4-oxadiazol-2-yl]-4-quinoline carboxamide, 2-(4-chlorophenyl)-N-[5-(2-furyl)-1,3,4-oxadiazol-2-yl]-4-quinoline carboxamide, N-[5-(2-furyl)-1,3,4-oxadiazol-2-yl]-2-(4-methylphenyl)-4-quinoline carboxamide, 2-(4-difluoromethoxyphenyl)-N-[5-(2-furyl)-1,3,4-oxadiazol-2-yl]-4-quinoline carboxamide, N-[5-(2-furyl)-1,3,4-oxadiazol-2-yl]-2-(4-hydroxyphenyl)-4-quinoline carboxamide, N-[5-(2-furyl)-1,3,4-oxadiazol-2-yl]-2-(4-methoxyphenyl)-4-quinoline carboxamide, N-[5-(2-furyl)-1,3,4-oxadiazol-2-yl]-2-(4-nitrophenyl)-4-quinoline carboxamide, 2-(4-tert-butoxycarbonylphenyl)-N-[5-(2-furyl)-1,3,4-oxadiazol-2-yl]-4-quinoline carboxamide, N-[5-(2-furyl)-1,3,4-oxadiazol-2-yl]-2-(2,4-dimethylphenyl)-4-quinoline carboxamide, N-[5-(2-furyl)-1,3,4-oxadiazol-2-yl]-2-(3,4-dimethoxyphenyl)-4-quinoline carboxamide, N-[5-(2-furyl)-1,3,4-oxadiazol-2-yl]-2-(3,4-methylenedioxyphenyl)-4-quinoline carboxamide, N-[5-(2-furyl)-1,3,4-oxadiazol-2-yl]-2-(1-naphthyl)-4-quinoline carboxamide, N-[5-(2-furyl)-1,3,4-oxadiazol-2-yl]-2-(6-methoxy-2-naphthyl)-quinoline carboxamide, N-[5-(2-furyl)-1,3,4-oxadiazol-2-yl]-2-(5-methyl-2-furyl)-4-quinoline carboxamide, N-[5-(2-furyl)-1,3,4-oxadiazol-2-yl]-2-phenyl-6-trifluoromethoxy-4-quinoline carboxamide, N-[5-(5-nitro-2-furyl)-1,3,4-oxadiazol-2-yl]-2-phenyl-4-quinoline carboxamide, N-[5-(2-furyl)-1,3,4-oxadiazol-2-yl]-6-(4-hydroxyphenyl)-2-phenyl-4-quinoline carboxamide, N-[5-(2-furyl)-1,3,4-oxadiazol-2-yl]-6-(3-thienyl)-2-phenyl-4-quinoline carboxamide, N-[5-(2-furyl)-1,3,4-oxadiazol-2-yl]-6-(3-pyridyl)-2-phenyl-4-quinoline carboxamide, N-(5-phenyl-1,3,4-oxadiazol-2-yl)-2-phenyl-6-trifluoromethoxy-4-quinoline carboxamide, N-[5-(2-chlorophenyl)-1,3,4-oxadiazol-2-yl]-2-phenyl-6-trifluoromethoxy-4-quinoline carboxamide, N-[5-(4-methoxyphenyl)-1,3,4-oxadiazol-2-yl]-2-phenyl-6-trifluoromethoxy-4-quinoline carboxamide, N-[5-(5-chloro-2-thienyl)-1,3,4-oxadiazol-2-yl]-2-phenyl-4-quinoline carboxamide, N-[5-(4-methoxyphenyl)-1,3,4-oxadiazol-2-yl]-2-phenyl-4-quinoline carboxamide and N-(5-phenyl-1,3,4-oxadiazol-2-yl)-2-(2-thienyl)-4-quinoline carboxamide, with N-[5-(2-furyl)-1,3,4-oxadiazol-2-yl]-2-phenyl-6-trifluoromethoxy-4-quinoline carboxamide being more preferable.

Examples of the pharmacologically acceptable salt of the quinoline carboxamide derivative of formula (I) include pharmacologically acceptable acid addition salts, metal salts, ammonium salts, organic amine addition salts and amino acid addition salts. Examples of the pharmacologically acceptable acid addition salt include salts of inorganic acids such as hydrochloric acid, hydrobromic acid, sulfuric acid, nitric acid, phosphoric acid and boric acid; and organic acids such as carboxylic acids such as formic acid, acetic acid, propionic acid, fumaric acid, malonic acid, succinic acid, maleic acid, tartaric acid, citric acid and benzoic acid, sulfonic acids such as methanesulfonic acid and p-toluenesulfonic acid, and amino acids such as glutamic acid and aspartic acid. Examples of the pharmacologically acceptable metal salt include salts of alkali metals such as lithium, sodium and potassium; salts of alkali earth metals such as magnesium and calcium; and metals such as aluminum and zinc, examples of the pharmacologically acceptable ammonium salt include salts of ammonium, tetramethylammonium and the like, examples of the pharmacologically acceptable organic amine salt include salts of triethylamine, piperidine, morpholine, toluidine and the like, and examples of the pharmacologically acceptable amino acid addition salt include addition salts of lysine, glycine, phenylalanine and the like.

A quinoline carboxamide derivative of formula (I) or a salt thereof according to the present invention is disclosed as a STAT3 inhibitor in JP-B-5650529 (Patent Literature 1), and can be manufactured by the method described in this publication. The quinoline carboxamide derivative or a salt thereof is known to exhibit an antitumor effect by inhibiting formation of a dimer of STAT3.

A cancer molecular target drug of the present invention is an agent developed for the purpose of not only suppressing growth of a primary tumor but also suppressing metastasis of the tumor by targeting a molecule, which is involved in growth, infiltration and metastasis of tumor cells, to suppress growth of tumor cells and inhibit a tumor progression process.

The cancer molecular target drug of the present invention comprises one or more selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor (hereinafter, referred to as a "cancer molecular target drug of the present invention").

Here, the "ALK inhibitor" inhibits tyrosine kinase of anaplastic lymphoma kinase (ALK).

The "EGFR inhibitor" inhibits the T790M genetic mutation and the activation mutation of EGFR (epidermal growth factor receptor).

The "multi kinase inhibitor" inhibits a plurality of tyrosine kinases such as RAF involved in tumor cell growth and angiogenesis, VEGFR (vascular endothelial growth factor), PDGFR (platelet-derived growth factor receptor) and RET (rearranged during transfection).

The "HER2/EGFR inhibitor" inhibits both EGFR and HER2 (EGFR2) of the EGFR family.

The "mTOR inhibitor" inhibits mTOR (mammalian target of rapamycin).

The "BRAF inhibitor" inhibits the kinase activity of mutant BRAF (V600E, V600K and V600D mutation positive).

The "MEK inhibitor" inhibits the kinase activity of MEK (mitogen-activated extracellular signal-regulated kinase) 1/MEK2.

The "BCR-ABL inhibitor" inhibits the tyrosine kinase of Bcr-Abl (breakpoint cluster region-abelson), KIT, PDGFR.

Specifically, examples of the ALK inhibitor include crizotinib, alectinib, ceritinib, lorlatinib, brigatinib and entrectinib; examples of the EGFR include osimertinib, gefitinib, erlotinib, afatinib, dacomitinib, cetuximab and panitumumab;

examples of the multi kinase inhibitor include sorafenib, vandetanib, lenvatinib, regorafenib, sunitinib, axitinib, pazopanib, cabozantinib and nintedanib; examples of HER2/EGFR inhibitor include lapatinib; examples of mTOR inhibitor include everolimus, temsirolimus, GDC-0980 (RG7422), AZD2014, PI-103, KU-0063794, AZD8055, GSK1059615, OSI-027, PF-04691502, PF-05212384 (PKI-587), WAY-600, GSK2126458, PP242, WYE-125132, WYE-687, PP-121, Torin 2, Torin 1 and INK 128; examples of the BRAF inhibitor include dabrafenib, vemurafenib, encorafenib; examples of the MEK inhibitor include trametinib and binimetinib; and examples of the BCR-ABL inhibitor include imatinib, dasatinib, bosutinib, ponatinib and nilotinib. Preferred cancer molecular target drug include alectinib, crizotinib, ceritinib, osimertinib, sorafenib, vandetanib, lenvatinib, lapatinib, everolimus, dabrafenib, trametinib, imatinib and dasatinib.

Of these cancer molecular target drugs, ALK inhibitors, EGFR inhibitors, multi kinase inhibitors, mTOR inhibitors, BRAF inhibitors, MEK inhibitors and BCR-ABL inhibitors are preferable, and ALK inhibitors and multi kinase inhibitors are preferable, from the viewpoint of the effect of use in combination with a quinoline carboxamide derivative of formula (I) or a salt thereof. The cancer molecular target drug is preferably alectinib, crizotinib, ceritinib, osimertinib, sorafenib, vandetanib, lenvatinib, everolimus, dabrafenib, trametinib, imatinib or dasatinib, more preferably alectinib, crizotinib, ceritinib, sorafenib or vandetanib.

As in Examples described below, administration of a quinoline carboxamide derivative of formula (I) or a salt thereof in combination with the cancer molecular target drug of the present invention exhibits a significantly higher cell injury activity and antitumor effect, compared with single-drug administration. For the cell injury activity, the synergetic effect is 0.9 or less in terms of a combination index (CI) which is a combination effect value calculated by a median-effect method (Pharmacol Rev 58: 621-681, 2006) using combination effect analyzing software CalcuSyn (HULINKS).

Therefore, a combination of a quinoline carboxamide derivative of formula (I) or a salt thereof and the cancer molecular target drug of the present invention is useful as an antitumor agent. A quinoline carboxamide derivative of formula (I) or a salt thereof is useful as an antitumor agent which is administered in combination with the cancer molecular target drug of the present invention. A quinoline carboxamide derivative of formula (I) or a salt thereof is useful as an antitumor effect enhancer for the cancer molecular target drug of the present invention, and the cancer molecular target drug of the present invention is useful as an antitumor effect enhancer for a quinoline carboxamide derivative of formula (I) or a salt thereof.

Examples of cancers which can be treated by the antitumor agent of the present invention include, without limitation, non-small cell lung cancer, tongue cancer, thyroid gland cancer, hepatocyte cancer, breast cancer, ovary cancer, uterine body cancer, melanoma and leukemia, with non-small cell lung cancer, tongue cancer, thyroid gland cancer, hepatocyte cancer, ovary cancer and uterine body cancer being preferable.

The antitumor agent obtained by combining a quinoline carboxamide derivative of formula (I) or a salt thereof with the cancer molecular target drug according to the present invention may be one obtained by formulating effective amounts of a quinoline carboxamide derivative of formula (I) above or a salt thereof and the cancer molecular target drug as a combination preparation at an appropriate combination ratio into one dosage form (one-dosage form), or one obtained by formulating agents containing respective effective amounts of the above-described ingredients so that the above-described ingredients can be separately used at the same time or at intervals (two-dosage form).

The administration form of the preparation is not limited, and can be appropriately selected according to the treatment purpose. Specific examples thereof include oral preparations (e.g. tablets, coated tablets, powders, granules, capsules and solutions), injections, suppositories, patches and ointments. A quinoline carboxamide derivative of formula (I) or a salt thereof and the cancer molecular target drug may be in a different administration forms or in the same administration form.

A preparation containing a quinoline carboxamide derivative of formula (I) or a salt thereof in the present invention and/or the cancer molecular target drug of the present invention can be prepared by a usual known method using a pharmacologically acceptable carrier. Examples of the carrier include various carriers which are commonly used in usual agents, for example excipients, binders, disintegrants, lubricants, diluents, solubilizing agents, suspending agents, tonicity agents, pH adjusters, buffers, stabilizers, colorants, flavor improving agents and odor improving agents.

Examples of the excipient include lactose, sucrose, sodium chloride, glucose, maltose, mannitol, erythritol, xylitol, maltitol, inositol, dextran, sorbitol, albumin, urea, starch, calcium carbonate, kaolin, crystalline cellulose, silicic acid, methylcellulose, glycerin, sodium alginate, gum arabic and mixtures thereof. Examples of the lubricant include purified talc, stearates, borax, polyethylene glycol and mixtures thereof. Examples of the binder include simply syrup, glucose solution, starch solution, gelatin solution, polyvinyl alcohol, polyvinyl ether, polyvinyl pyrrolidone, carboxymethylcellulose, shellac, methylcellulose, ethylcellulose, water, ethanol, potassium phosphate and mixtures thereof. Examples of the disintegrant include dry starch, sodium alginate, powdered agar, powdered laminaran, sodium hydrogencarbonate, calcium carbonate, polyoxyethylene sorbitan fatty acid esters, sodium lauryl sulfate, monoglyceride stearate, starch, lactose and mixtures thereof. Examples of the diluent include water, ethyl alcohol, macrogol, propylene glycol, ethoxylated isostearyl alcohol, polyoxylated isostearyl alcohol, polyoxyethylene sorbitan fatty acid esters and mixtures thereof. Examples of the stabilizer include sodium pyrosulfite, ethylenediaminetetraacetic acid, thioglycolic acid, thiolactic acid and mixtures thereof. Examples of the tonicity agent include sodium chloride, boric acid, glucose, glycerin and mixtures

thereof. Examples of the pH adjuster and the buffer include sodium citrate, citric acid, sodium acetate, sodium phosphate and mixtures thereof. Examples of the soothing agent include procaine hydrochloride, lidocaine hydrochloride and mixtures thereof.

The amounts of a quinoline carboxamide derivative of formula (I) or a salt thereof and the cancer molecular target drug of the present invention blended in the preparation can be appropriately set, and in general, the amount of a quinoline carboxamide derivative of formula (I) or a salt thereof in the preparation is from 0.001 to 5,000 mg, preferably from 0.1 to 1,000 mg, more preferably from 1 to 500 mg. The amount of the cancer molecular target drug is appropriately set within a range which is acceptable for each agent. For example, the amount of the cancer molecular target drug is from 150 to 600 mg in the case of alectinib, or from 200 to 800 mg in the case of sorafenib.

When the antitumor agent of the present invention is used as a kit, the antitumor agent can be designed so that agents each formulated as above and containing a quinoline carboxamide derivative of formula (I) or a salt thereof and the cancer molecular target drug of the present invention are separately packaged, and each pharmaceutical preparation is taken out from each package and used. Each pharmaceutical preparation can be packaged in a form suitable for combined administration each time.

The dosage amount of a quinoline carboxamide derivative of formula (I) or a salt thereof in the present invention and the cancer molecular target drug of the present invention is not limited, and is appropriately set according to the age of a patient, the cancer species, the disease stage, whether metastasis occurs or not, the treatment history, whether other antitumor agents are present or not, or the like, as long as the quinoline carboxamide derivative of formula (I) or a salt thereof and the cancer molecular target drug of the present invention can synergistically exhibit an antitumor effect to effectively treat cancer. The amount of a quinoline carboxamide derivative of formula (I) or a salt thereof is preferably from 0.001 to 5,000 mg/day, preferably from 0.1 to 1,000 mg/day, more preferably 1 to 500 mg. For example, the amount of the cancer molecular target drug is from 150 to 600 mg in the case of alectinib, or from 200 to 800 mg in the case of sorafenib.

The administration order and the administration interval of a quinoline carboxamide derivative of formula (I) or a salt thereof in the present invention and the cancer molecular target drug are not limited as long as a synergetic effect can be obtained. When the antitumor agent is used as a kit, single preparations may be administered at the same time or at intervals.

Examples

<Materials and Experimental Method>

1. Cell culture

NCI-H2228, NCI-H1975, SK-BR-3, Hep3B, MCF-7, Caov-3, A2058, K562 and SUP-B15 were purchased from American Type Culture Collection, SAS and HuH-7 were purchased from National Institutes of Biomedical Innovation, Health and Nutrition, and K1, FTC-133 and Ishikawa were purchased European Collection of Authenticated Cell Cultures. The cells were subcultured under conditions of 5% $CO_2$ and 37°C in culture media containing 100 U/mL penicillin, 100 $\mu$g/mL streptomycin (Thermo Fisher Scientific, Cat. No. 15140-122) as shown in Table 1, and were used for experiments. Fetal bovine serum (hereinafter, abbreviated as FBS) and MCDB 105 culture medium were purchased from Sigma Aldrich (Cat. Nos. 172012 and 117-500), and RMPI1640 culture medium, DMEM medium, Ham's F-12 medium, MEM medium and IMDM culture medium were purchased Thermo Fisher Scientific (Cat. Nos. A1049101, 11995-065, 11765-054, 11095-080 and 12440-053).

[Table 1]

| Cell name | Type of cells | Culture solution |
|---|---|---|
| NCI-H2228 | Human non-small cell lung cancer cells | 10% FBS-containing RPMI1640 medium |
| NCI-H1975 | Human non-small cell lung cancer cells | 10% FBS-containing RPMI1640 medium |
| SAS | Human tongue cancer cells | 10% FBS-containing RPMI1640 medium |
| K1 | Human thyroid gland cancer cells | 10% FBS-containing DMEM: Ham's F-12: MCDB105 (2: 1 : 1) medium |
| FTC-133 | Human thyroid gland cancer cells | 10% FBS-containing DMEM: Ham's F-12 (1 : 1) medium |

(continued)

| Cell name | Type of cells | Culture solution |
|---|---|---|
| SK-BR-3 | Human breast cancer cells | 10% FBS-containing RPMI1640 medium |
| Hep3B | Human hepatocyte cancer cells | 10% FBS-containing MEM medium |
| HuH-7 | Human hepatocyte cancer cells | 10% FBS-containing DMEM medium |
| MCF-7 | Human breast cancer cells | 10% FBS-containing DMEM medium |
| Caov-3 | Human ovary cancer cells | 10% FBS-containing DMEM medium |
| Ishikawa | Human uterine body cancer cells | 10% FBS, 1% Glutamine-containing MEM medium |
| A2058 | Human melanoma cells | 10% FBS-containing DMEM medium |
| K562 | Human chronic myelocytic leukemia cells | 10% FBS-containing RPMI1640 medium |
| SUP-B15 | Acute lymphatic leukemia cells | 20% FBS, 0.05 mM 2-mercaptoethanol-containing IMDM medium |

2. Agents

N-[5-(2-furyl)-1,3,4-oxadiazol-2-yl]-2-phenyl-6-trifluoromethoxy-4-quinoline carboxamide (hereinafter, referred to as "STX-1159") was synthesized in accordance with the method described in Patent Literature 1.

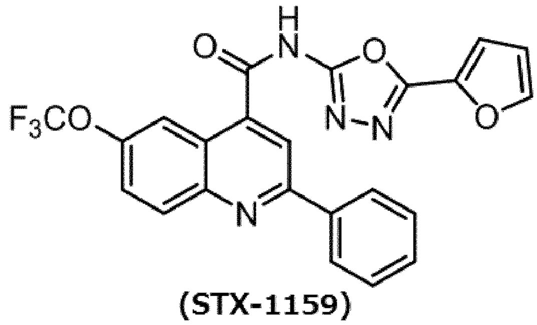

**(STX-1159)**

Crizotinib, everolimus and trametinib were purchased from LC Laboratories (Cat. Nos. C-7900, E-4040, V-2800 and T-8123). Alectinib, osimertinib and lenvatinib were purchased from Selleckchem.com (Cat. Nos. S2762, S7297 and S1164), and ceritinib was purchased from Active Biochem (Cat. No, A-1189). Sorafenib was purchased from Cayman Chemical (Cat. No. 10009644), vandetanib, lapatinib and dabrafenib were purchased from Santa Cruz Biotechnology (Cat. Nos. sc-220364, sc-202205 and sc-364477). Imatinib was purchased from Cell Signaling Technology (Cat. No. 9084), and dasatinib was purchased from Bio Vision (Cat. No. 1586). All the agents were soluble in dimethylsulfoxide (hereinafter, abbreviated as DMSO).

Example 1: Evaluation of agent combination effect

(1) Setting of reference dose

The cells were inoculated on a 96-well plate to the density shown in Table 2, and were cultured under conditions of 5% $CO_2$ and 37°C. The agents were singly added one day after the cells were inoculated. To the control group was added DMSO, so as to be a final concentration of 0.05%. Subsequently, the cells were cultured under conditions of 5% $CO_2$ and 37°C for the time shown in Table 2, and the number living cells was evaluated by WST-8 assay. That is, 10 μL of a WST-8 kit solution (Kishida Chemical Co., ltd, Cat. No. 260-96162) was added to each well, and the cells were cultured under conditions of 5% $CO_2$ and 37°C for 1 to 2 hours. The absorbance of water-soluble formazan generated by enzymatic activity of mitochondria in cells was measured at 450 nm using a microplate reader (Molecular Devices, Model: SpectraMax Plus). This was evaluated as the viable cell count to calculate a 50% cell growth inhibitory concentration (hereinafter, abbreviated as $IC_{50}$). The reference dose of each agent was set based on $IC_{50}$ (Table 3) .

[Table 2]

| Cell density and culture time | | |
|---|---|---|
| Cell name | Cell density | Culture time after addition of agent |
| NCI-H2228 | 2500 cells/well | 72 hours |
| NCI-H1975 | 2500 cells/well | 72 hours |
| SAS | 2500 cells/well | 48 hours |
| K1 | 2500 cells/well | 72 hours |
| FTC-133 | 2500 cells/well | 72 hours |
| Hep3B | 2500 cells/well | 72 hours |
| HuH-7 | 2500 cells/well | 72 hours |
| SK-BR-3 | 2500 cells/well | 72 hours |
| MCF-7 | 2500 cells/well | 72 hours |
| Caov-3 | 2500 cells/well | 72 hours |
| Ishikawa | 2500 cells/well | 72 hours |
| A2058 | 2500 cells/well | 72 hours |
| K562 | 2500 cells/well | 48 hours |
| SUP-B15 | 7500 cells/well | 72 hours |

[Table 3]

| Reference dose | | |
|---|---|---|
| Cell name | Concentration of STX-1159 | Concentration of combination agent |
| NCI-H2228 | 10 μM | Crizotinib 1 μM |
| | | Alectinib 0.1 μM |
| | | Ceritinib 0.5 μM |
| NCI-H1975 | 2 μM | Osimertinib 1 μM |
| SAS | 0.5 μM | Sorafenib 3 μM |
| K1 | 7 μM | Sorafenib 10 μM |
| | | Vandetanib 15 μM |
| FTC-133 | 2.5 μM | Sorafenib 8 μM |
| | | Vandetanib 15 μM |
| Hep3B | 1 μM | Lenvatinib 0.5 μM |
| HuH-7 | 1 μM | Lenvatinib 1 μM |
| SK-BR-3 | 1 μM | Lapatinib 0.05 μM |
| MCF-7 | 2 μM | Everolimus 0.0015 μM |
| Caov-3 | 2 μM | Everolimus 15 μM |
| Ishikawa | 1 μM | Everolimus 0.0005 μM |
| A2058 | 0.7 μM | Dabrafenib 50 μM |
| | | Trametinib 0.005 μM |

(continued)

| Reference dose | | |
|---|---|---|
| Cell name | Concentration of STX-1159 | Concentration of combination agent |
| K562 | 1.5-2 μM | Imatinib 0.2 - 0.3 μM |
| | | Dasatinib 0.0003 - 0.00035 μM |
| SUP-B15 | 1 - 1.5 μM | Imatinib 0.35 - 0.7 μM |
| | | Dasatinib 0.03 μM |

(2) Evaluation of combination effect by median-effect method

The cells were inoculated on a 96-well plate so as to be the density shown in Table 2, and were cultured under conditions of 5% $CO_2$ and 37°C. One day after the cells were inoculated, for single groups, STX-1159 and the agents were each added so as to be a final concentration which is 1/4 times, 1/2 times, 1 time, 2 times or 4 times the reference dose, and for combination groups, STX-1159 and the agents were added so as to be a final concentration where STX-1159 and the agents combined were each 1/4 times, 1/2 times, 1 time, 2 times or 4 times the reference dose. To the control group was added DMSO, so as to be a final concentration of 0.1%. Subsequently, the cells were cultured under conditions of 5% $CO_2$ and 37°C for the time shown in Table 2, and the number living cells was measured by WST-8 assay. The fraction affected (hereinafter, abbreviated as fa) was calculated from the following expression:

fa = 1 - (viable cell count in agent exposure group)/(viable cell count in control group).

By a median-effect method using combination effect analyzing software CalcuSyn (HULINKS), the combination index (hereinafter, abbreviated as CI) was calculated from fa obtained when STX-1159 and the agents were exposed alone or in combination. Evaluation of the combination effect was based on grading by CI in Table 4. In calculation of CI, the average of the values from two or more experiments was employed.

[Table 4]

| Grading of combination effect by CI | |
|---|---|
| CI | Combination effect |
| CI ≤ 0.1 | Very intense synergetic effect |
| 0.1 < CI ≤ 0.3 | Intense synergetic effect |
| 0.3 < CI ≤ 0.7 | Synergetic effect |
| 0.7 < CI ≤ 0.85 | Moderate synergetic effect |
| 0.85 < CI ≤ 0.9 | Light synergetic effect |
| 0.9 < CI ≤ 1.1 | Substantially additive effect |
| 1.1 < CI ≤ 1.2 | Light antagonistic action |
| 1.2 < CI ≤ 1.45 | Moderate antagonistic action |
| 1.45 < CI ≤ 3.3 | Antagonistic action |
| 3.3 < CI ≤ 10 | Intense antagonistic action |
| 10 < CI | Very intense antagonistic action |

(3) Results

The results of evaluating the combination effect of STX-1159 and the agents by the median-effect method showed that use of STX-1159 in combination with crizotinib, alectinib, ceritinib, osimertinib, sorafenib, vandetanib, lenvatinib, lapatinib, everolimus, dabrafenib, trametinib, imatinib or dasatinib exhibited a synergetic effect (Table 5). These results indicated that combinations of STX-1159 with any of these agents were all effective as a combination drug therapy.

[Table 5]

| Results of evaluating combination effect | | | |
|---|---|---|---|
| Cell name | Combination agent | CI | Combination effect |
| NCI-H2228 | Crizotinib | 0.50 | Synergetic effect |
| | Alectinib | 0.57 | Synergetic effect |
| | Ceritinib | 0.34 | Synergetic effect |
| NCI-H1975 | Osimertinib | 0.81 | Moderate synergetic effect |
| SAS | Sorafenib | 0.56 | Synergetic effect |
| K1 | Sorafenib | 0.77 | Moderate synergetic effect |
| | Vandetanib | 0.69 | Synergetic effect |
| FTC-133 | Sorafenib | 0.85 | Light synergetic effect |
| | Vandetanib | 0.75 | Moderate synergetic effect |
| Hep3B | Lenvatinib | 0.75 | Moderate synergetic effect |
| HuH-7 | Lenvatinib | 0.85 | Moderate synergetic effect |
| SK-BR-3 | Lapatinib | 0.88 | Light synergetic effect |
| MCF-7 | Everolimus | 0.73 | Moderate synergetic effect |
| Caov-3 | Everolimus | 0.90 | Light synergetic effect |
| Ishikawa | Everolimus | 0.89 | Light synergetic effect |
| A2058 | Dabrafenib | 0.73 | Moderate synergetic effect |
| | Trametinib | 0.78 | Moderate synergetic effect |
| K562 | Imatinib | 0.83 | Moderate synergetic effect |
| | Dasatinib | 0.78 | Moderate synergetic effect |
| SUP-B15 | Imatinib | 0.85 | Moderate synergetic effect |
| | Dasatinib | 0.71 | Moderate synergetic effect |

Example 2: Suppression of signaling pathway forming molecules of STAT3 and ALK by use of STX-1159 in combination with alectinib

(1) Evaluation by western blotting method

For the purpose of confirming that a combined use of STX-1159 and alectinib synergistically exhibiting an inhibitory action on growth of NCI-H2228 cells inhibited STAT3 or ALK in cells, actions of both the agents on the protein amounts of phosphorylated STAT3, phosphorylated ALK, survivin and c-myc in NCI-H2228 cells was examined by a western blotting method.

NCI-H2228 cells were inoculated on a 6-well plate so as to be $2 \times 10^5$ cells/well, and cultured under conditions of 5% $CO_2$ and 37°C. One day after the cells were inoculated, STX-1159 and alectinib were added alone or in combination so as to be final concentrations of 5 $\mu$M for STX-1159 and 0.01 or 0.1 $\mu$M for alectinib. To the control group was added DMSO, so as to be a final concentration of 0.1%. After the agents were added, the cells were cultured under conditions of 5% $CO_2$ and 37°C for 24 hours, and then collected. The cells were washed once with ice-cooled PBS, and RIPA buffer (25 mM Tris-HCl, pH 7.6, 1% NP-40, 1% sodium deoxycholate, 0.1% SDS, 0.15 M NaCl) with a protease inhibitor cocktail (nacalai tesque, Cat. No. 25955-11) and a phosphatase cocktail (nacalai tesque, Cat. No. 07574) was added to lyse the cells. The resulting lysate was subjected to electrophoresis, and proteins in acrylamide gel were transcribed to Immobilon PVDF membrane using a semidry-type transcription apparatus. After the transcription, the membrane was blocked, and immersed in a primary antibody (anti-phosphorylation STAT3 (Y705) antibody: Cell Signaling technology,

Cat. No. CST9131, anti-STAT3 antibody: Cell Signaling Technology, Cat. No. CST4904, phosphorylated ALK (Y1278/Y1282/Y12283)antibody: Cell Signaling Technology, Cat. No. CST3983, anti-ALK antibody: Cell Signaling Technology, Cat. No. CST3633, anti-survivin antibody: R&D Systems, Cat. No. AF886, anti-c-myc antibody: Cell Signaling Technology, Cat. No. CST9402 and anti-β-actin antibody: Sigma Aldrich, Cat. No. A5316) solution at 4°C overnight. Further, the membrane was immersed in a secondary antibody solution at room temperature for 1 hour, and target proteins on the membrane were then detected using ECL select (GE Healthcare Japan, Cat. No. RPN2235).

(2) Results

Figure 1 shows the results of western blotting.

Whether use of STX-1159 in combination with alectinib inhibited STAT3 or ALK in cells was examined. Use of STX-1159 alone reduced the amounts of phosphorylated STAT3, survivin and c-myc, and use of alectinib alone reduced the amounts of phosphorylated ALK, phosphorylated STAT3 and c-myc. The amounts of phosphorylated STAT3 and c-myc (downstream factors of the STAT pathway and the ALK pathway) were more reduced by use of both the agents in combination than by use of each single agent alone. This result indicated that use of combination of STK-1159 with alectinib exhibited a cell growth inhibitory action in a synergistic manner by duplicately inhibiting the STAT3 pathway.

Example 3: Evaluation of combination effect of STX-1159 and mTOR inhibitor

(1) Setting of reference dose

Human breast cell line MDA-MB-231 cells or MDA-MB-468 cells (in the case of rapamycin) were inoculated on a 96-well plate to 2.5 × $10^3$/100 μL/well or 1.0 × $10^3$/100 μL/well, and cultured under conditions of 5% $CO_2$ and 37°C. One day after the cells were inoculated, the agents shown in Table 7 were singly added. To the control group was added DMSO, so as to be a final concentration of 0.05%. Subsequently, the cells were cultured under conditions of 5% $CO_2$ and 37°C for 48 hours, and the viable cell count was then evaluated by WST-8 assay. That is, 10 μL of a WST-8 kit solution (Kishida Chemical Co., ltd, Cat. No. 260-96162) was added to each well, and the cells were cultured under conditions of 5% $CO_2$ and 37°C for 1 to 2 hours. The absorbance of water-soluble formazan generated by enzymatic activity of mitochondria in cells was measured at 450 nm using a microplate reader (Molecular Devices, Model: SpectraMax Plus). This was evaluated as the viable cell count to calculate a 50% cytostatic concentration (hereinafter, abbreviated as $IC_{50}$). The reference dose of each agent was set based on $IC_{50}$ (Table 6) .

[Table 6]

| Reference dose | | |
|---|---|---|
| Concentration of STX-1159 | Concentration of combination agent | |
| 2.5-40 μM | GDC-0980(RG7422) | 0.094-1.5 μM |
| 2.5-40 μM | AZD2014 | 0.125-2 μM |
| 2.5-40 μM | PI-103 | 0.031-0.5 μM |
| 2.5-40 μM | KU-0063794 | 0.625-10 μM |
| 2.5-40 μM | AZD8055 | 0.183-3 μM |
| 2.5-40 μM | GSK1059615 | 0.625-10 μM |
| 2.5-40 μM | OSI-027 | 0.625-10 μM |
| 2.5-40 μM | PF-04691502 | 0.125-2 μM |
| 2.5-40 μM | PF-05212384(PKI-587) | 0.016-0.25 μM |
| 2.5-40 μM | WAY-600 | 0.625-10 μM |
| 2.5-40 μM | GSK2126458 | 0.063-1 μM |
| 2.5-40 μM | PP242 | 0.063-1 μM |
| 2.5-40 μM | WYE-125132 | 0.063-1 μM |
| 2.5-40 μM | WYE-687 | 0.188-3 μM |
| 2.5-40 μM | PP-121 | 0.063-1 μM |
| 2.5-40 μM | Torin 2 | 0.016-0.250 μM |

(continued)

| Reference dose | | |
|---|---|---|
| Concentration of STX-1159 | Concentration of combination agent | |
| 2.5-40 μM | Torin 1 | 0.188-3 μM |
| 2.5-40 μM | INK 128 | 0.016-0.250 μM |
| 0.25-4 μM | Rapamycin | 0.0003-0.005 μM |

(2) Experimental method for WST-8 assay

Human breast cell line MDA-MB-231 cells or MDA-MB-468 cells (in the case of rapamycin) were inoculated on a 96-well plate so as to be 2.5 × $10^3$/100 μL/well or 1.0 × $10^3$/100 μL/well, respectively. The cells were incubated in a $CO_2$ incubator at 37°C. After 24 hours, the supernatant was discarded, and 80 μL of a culture medium for assay was added. 10 μL of an mTOR inhibitor solution diluted stepwise (shown in Table 7) was added, and 10 μL of a STX-1159 solution diluted stepwise was then added. The mixture was left standing in a $CO_2$ incubator at 37°C for 48 hours, the supernatant was then removed, and the mixture was washed once with 100 μL of PBS(-). A WST-8 solution diluted by 10 times with the medium for assay was added at 100 μL/well, and the absorbance was measured at a wavelength of 450 nm using a plate reader. CI was calculated from the measured value.

(3) Calculation of CI value

The combination effect of STX-1159 and the mTOR inhibitor was analyzed using Calcusyn software (BIOSOFT, Cambridge, UK). For grading of the combination effect by CI, the same criteria as in Table 4 in Example 1 were used. Table 7 shows the results.

(4) Results

Use of STX-1159 in combination with the mTOR inhibitor shown in Table 7 exhibited a synergetic effect (Table 7). The result indicated that combinations of STX-1159 with any of these agents were all effective as a combination drug therapy.

[Table 7]

| Combination agent | CI | Combination effect |
|---|---|---|
| GDC-0980(RG7422) | 0.86 | Light synergetic effect |
| AZD2014 | 0.51 | Synergetic effect |
| PI-103 | 0.60 | Synergetic effect |
| KU-0063794 | 0.36 | Synergetic effect |
| AZD8055 | 0.36 | Synergetic effect |
| GSK1059615 | 0.64 | Synergetic effect |
| OSI-027 | 0.49 | Synergetic effect |
| PF-04691502 | 0.73 | Moderate synergetic effect |
| PF-05212384(PKI-587) | 0.63 | Synergetic effect |
| WAY-600 | 0.64 | Synergetic effect |
| GSK2126458 | 0.63 | Synergetic effect |
| PP242 | 0.84 | Moderate synergetic effect |
| WYE-125132 | 0.40 | Synergetic effect |
| WYE-687 | 0.85 | Moderate synergetic effect |
| PP-121 | 0.88 | Light synergetic effect |

(continued)

| Combination agent | CI | Combination effect |
|---|---|---|
| Torin 2 | 0.66 | Synergetic effect |
| Torin 1 | 0.68 | Synergetic effect |
| INK 128 | 0.56 | Synergetic effect |
| Rapamycin | 0.79 | Moderate synergetic effect |

**Claims**

1. An antitumor agent comprising a combination of a quinoline carboxamide derivative of formula (I) or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

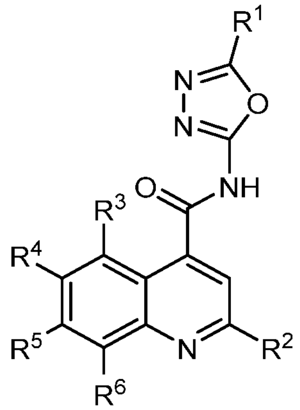

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

2. The antitumor agent according to claim 1, which is a kit comprising: an agent comprising a quinoline carboxamide derivative of formula (I) or a pharmacologically acceptable salt thereof; and an agent comprising one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor.

3. The antitumor agent according to claim 1, which is a combination preparation.

4. The antitumor agent according to any one of claims 1 to 3, wherein in formula (I), $R^1$ and $R^2$ are the same or different, and each represent a substituted or unsubstituted aryl group or a substituted or unsubstituted aromatic heterocyclic group.

5. The antitumor agent according to claim 4, wherein in formula (I), at least one of $R^3$, $R^4$, $R^5$ and $R^6$ is a group other than a hydrogen atom.

6. The antitumor agent according to any one of claims 1 to 5, wherein in $R^1$ and $R^2$ in formula (I), the aryl group is a phenyl group, and the aromatic heterocyclic group is a furyl group.

7. The antitumor agent according to any one of claims 1 to 5, wherein in formula (I), $R^1$ is a furyl group, and $R^2$ is a substituted or unsubstituted phenyl group.

8. The antitumor agent according to any one of claims 1 to 7, wherein at least one of $R^3$, $R^4$, $R^5$ and $R^6$ is a trifluor-

omethoxy group.

**9.** The antitumor agent according to any one of claims 1 to 7, wherein $R^4$ is a trifluoromethoxy group.

**10.** The antitumor agent according to any one of claims 1 to 9, wherein the quinoline carboxamide derivative of formula (I) is N-[5-(2-furyl)-1,3,4-oxadiazol-2-yl]-2-phenyl-6-trifluoromethoxy-4-quinoline carboxamide.

**11.** The antitumor agent according to any one of claims 1 to 10, wherein the cancer molecular target drug comprises one or more selected from the group consisting of crizotinib, alectinib, ceritinib, osimertinib, sorafenib, vandetanib, lenvatinib, lapatinib, everolimus, dabrafenib, trametinib, imatinib and dasatinib.

**12.** The antitumor agent according to any one of claims 1 to 10, wherein the cancer molecular target drug comprises one or more selected from the group consisting of crizotinib, alectinib, ceritinib, osimertinib, sorafenib, vandetanib, lenvatinib, everolimus, dabrafenib, trametinib, imatinib and dasatinib.

**13.** The antitumor agent according to any one of claims 1 to 12, wherein the cancer comprises one or more selected from the group consisting of non-small cell lung cancer, tongue cancer, thyroid gland cancer, hepatocyte cancer, breast cancer, ovary cancer, uterine body cancer, melanoma and leukemia.

**14.** An antitumor agent comprising a quinoline carboxamide derivative of formula (I) below or a pharmacologically acceptable salt thereof as an active ingredient, the antitumor agent being administered in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

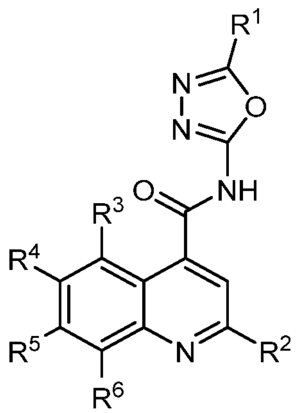

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**15.** An antitumor effect enhancer for one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, the antitumor effect enhancer comprising a quinoline carboxamide derivative of formula (I) below or a pharmacologically acceptable salt thereof as an active ingredient:

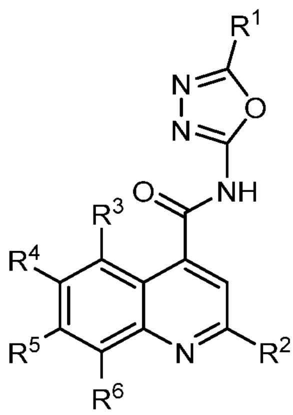

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**16.** A pharmaceutical composition comprising: a quinoline carboxamide derivative of formula (I) below or a pharmacologically acceptable salt thereof; and one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

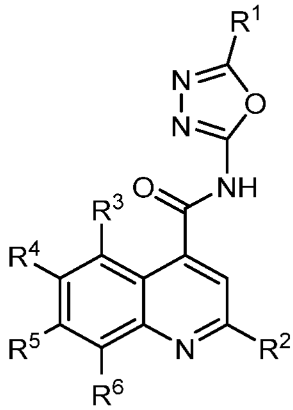

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**17.** A combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, for use in treating a tumor:

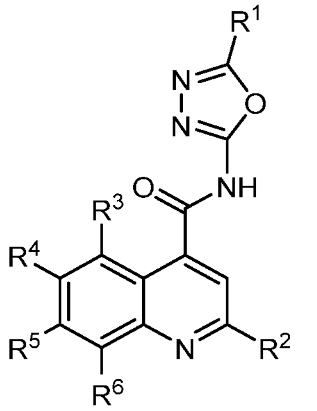

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**18.** A quinoline carboxamide derivative of formula (I) below or a pharmacologically acceptable salt thereof, for use in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, in treating a tumor:

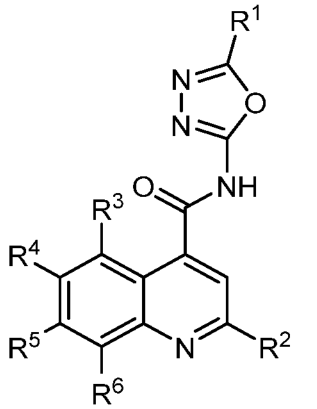

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**19.** A method for treating a tumor, comprising administering to a patient a therapeutically effective amount of a quinoline carboxamide derivative of formula (I) below or a pharmacologically acceptable salt thereof and one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

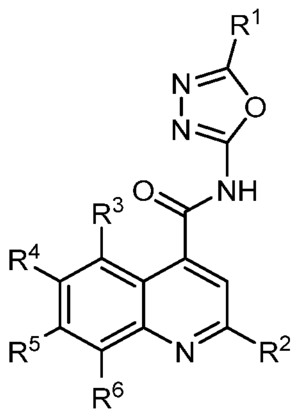

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**20.** Use of a combination of a quinoline carboxamide derivative of formula (I) below or a pharmacologically acceptable salt thereof with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, for manufacturing an antitumor agent:

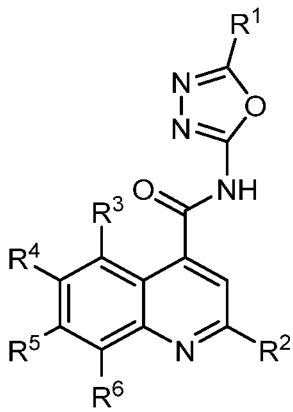

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**21.** Use of a quinoline carboxamide derivative of formula (I) below or a pharmacologically acceptable salt thereof, for manufacturing an antitumor agent which is administered in combination with one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

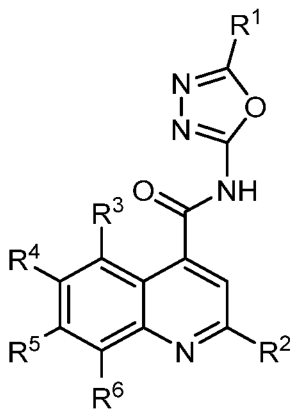

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**22.** A quinoline carboxamide derivative of formula (I) below or a pharmacologically acceptable salt thereof, for enhancing an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

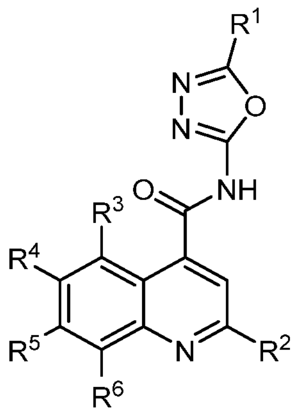

(I)

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**23.** A method for enhancing an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor, the method comprising a therapeutically effective amount of a quinoline carboxamide derivative of formula (I) below or a pharmacologically acceptable salt thereof to a patient:

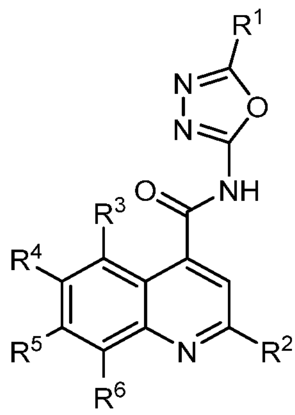

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

**24.** Use of a quinoline carboxamide derivative of formula (I) below or a pharmacologically acceptable salt thereof, for manufacturing an antitumor effect enhancer which enhances an antitumor effect of one or more cancer molecular target drugs selected from the group consisting of an ALK inhibitor, an EGFR inhibitor, a multi kinase inhibitor, a HER2/EGFR inhibitor, an mTOR inhibitor, a BRAF inhibitor, a MEK inhibitor and a BCR-ABL inhibitor:

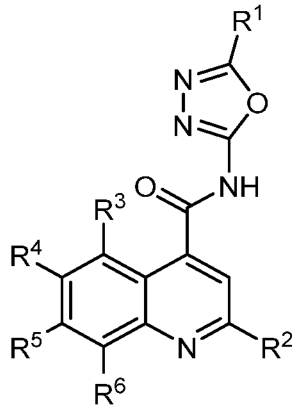

( I )

wherein $R^1$, $R^2$, $R^3$, $R^4$, $R^5$ and $R^6$ are the same or different, and each represent a hydrogen atom, a substituted or unsubstituted aryl group, a substituted or unsubstituted aromatic heterocyclic group, $COOR^7$ (wherein $R^7$ represents a substituted or unsubstituted alkyl group), or $OR^8$ (wherein $R^8$ represents a substituted or unsubstituted alkyl group).

[Figure 1]

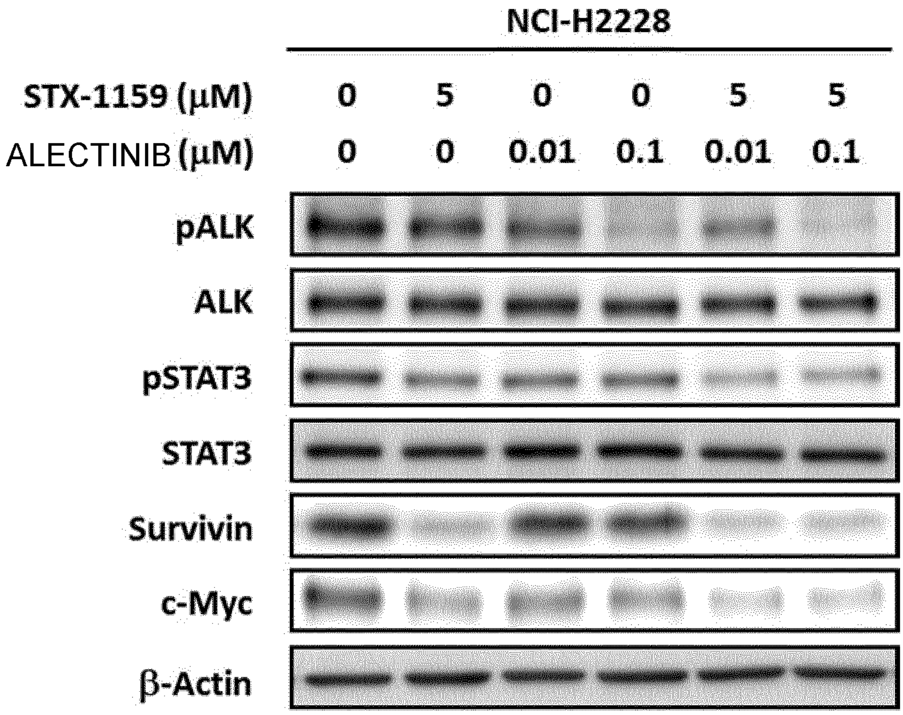
NCI-H2228
STX-1159 (μM) 0 5 0 0 5 5
ALECTINIB (μM) 0 0 0.01 0.1 0.01 0.1
pALK
ALK
pSTAT3
STAT3
Survivin
c-Myc
β-Actin

**INTERNATIONAL SEARCH REPORT**

International application No. PCT/JP2019/036363

A. CLASSIFICATION OF SUBJECT MATTER
Int.Cl. See extra sheet
According to International Patent Classification (IPC) or to both national classification and IPC

B. FIELDS SEARCHED
Minimum documentation searched (classification system followed by classification symbols)
Int.Cl. A61K31/4709, A61K31/436, A61K31/44, A61K31/4545, A61K31/47, A61K31/506, A61K31/517, A61K31/519, A61K31/5377, A61K45/00, A61P1/02, A61P1/16, A61P11/00, A61P15/00, A61P17/00, A61P35/00, A61P35/02, A61P43/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | MIYATA, Haruo et al., "Combination of a STAT3 inhibitor and an mTOR inhibitor against a temozolomide-resistant glioblastoma cell line", 2017, vol. 14 (1), pp. 83-91, abstract | 18, 22<br>1-17, 19-21, 23, 24 |
| X<br>A | MATSUNO, Kenji et al. "Identification of a New Series of STAT3 Inhibitors by Virtual Screening", ACS Med. Chem. Lett., 2010, vol. 1 (8), pp. 371-375, abstract | 18, 22<br>1-17, 19-21, 23, 24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 December 2019 (04.12.2019) | 07 January 2020 (07.01.2020) |
| Name and mailing address of the ISA/<br>Japan Patent Office<br>3-4-3, Kasumigaseki, Chiyoda-ku, | Authorized officer<br><br>Telephone No. |

Form PCT/ISA/210 (second sheet) (January 2015)

**INTERNATIONAL SEARCH REPORT**

International application No. PCT/JP2019/036363

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2010/004761 A1 (PHARMA IP GENERAL INCORPORATED ASSOCIATION) 14 January 2010, claims, paragraphs [0004], [0020], examples & US 2011/0172429 A1 (claims, paragraphs [0004], [0027], examples) | 18, 22<br>1-17, 19-21, 23, 24 |
| X<br>A | ISLAM, Rafiqul et al., "Facile Synthesis of 2-Phenylquinoline-4-Carboxamide Derivatives with Variant Structural Features", Heterocycles, 2014, vol. 89 (3), pp. 693-708, fig. 1, scheme 3 (compound 15a-c) | 18, 22<br>1-17, 19-21, 23, 24 |
| Y | WO 2017/076355 A1 (HUBEI BIOLOGICAL MEDICINE INDUSTRIAL TECHNOLOGY INSTITUTE CO., LTD.) 11 May 2017, abstract & JP 2018-532759 A & US 2018/0338973 A1 (abstract) | 1-10, 13-17, 19-21, 23, 24 |
| Y | JP 2017-530139 A (XUANZHU PHARMA CO., LTD.) 12 October 2017, abstract & US 2017/0240534 A1 (abstract) & WO 2016/050171 A1 | 1-10, 13-17, 19-21, 23, 24 |
| Y | JP 2015-510879 A (PFIZER INC.) 13 April 2015, abstract & US 2013/0252961 A1 (abstract) & WO 2013/132376 A1 | 1-10, 13-17, 19-21, 23, 24 |
| Y | JIAN, Kong et al., "YC-1 enhances the anti-tumor activity of sorafenib through inhibition of signal transducer and activator of transcription 3 (STAT3) in hepatocellular carcinoma", Molecular Cancer, 2014, vol. 13 (7), pp. 1-11, abstract, page 2, right column, paragraph [0001], page 9, right column, paragraph [0002] | 1-17, 19-21, 23, 24 |
| Y | HAO, Yang et al., "Ilexgenin A exerts anti-inflammation and anti-angiogenesis effects through inhibition of STAT3 and PI3K pathways and exhibits synergistic effects with Sorafenib on hepatoma growth", 2017, vol. 315, pp. 90-101, abstract, page 100, left column, paragraph [0001] | 1-17, 19-21, 23, 24 |
| Y | WEI-TIEN, Tai et al., "Dovitinib Induces Apoptosis and Overcomes Sorafenib Resistance in Hepatocellular Carcinoma through SHP-1-Mediated Inhibition of STAT3, Molecular Cancer Therapeutics", 2012, vol. 11 (2), pp. 452-463, abstract, page 462, left column, paragraph [0002] to right column, paragraph [0001] | 1-17, 19-21, 23, 24 |

Form PCT/ISA/210 (continuation of second sheet) (January 2015)

**INTERNATIONAL SEARCH REPORT**

International application No. PCT/JP2019/036363

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2018-504418 A (TYRNOVO LTD.) 15 February 2018, paragraph [0009] & US 2018/0028475 A1 & WO 2016/125169 A1 (page 3, paragraph [0002]) | 1-10, 13-17, 19-21, 23, 24 |
| X<br>A | ASHIZAWA, Tadashi et al., "Effect of the STAT3 inhibitor STX-0119 on the proliferation of cancer stem-like cells derived from recurrent glioblastoma", International Journal of Oncology, 2013, vol. 43 (1), pp. 219-227, abstract | 18, 22<br>1-17, 19-21, 23, 24 |

Form PCT/ISA/210 (continuation of second sheet) (January 2015)

Form PCT/ISA/210 (continuation of first sheet (2)) (January 2015)

**INTERNATIONAL SEARCH REPORT**

International application No.
PCT/JP2019/036363

CLASSIFICATION OF SUBJECT MATTER
A61K31/4709(2006.01)i, A61K31/436(2006.01)i, A61K31/44(2006.01)i, A61K31/4545(2006.01)i, A61K31/47(2006.01)i, A61K31/506(2006.01)i, A61K31/517(2006.01)i, A61K31/519(2006.01)i, A61K31/5377(2006.01)i, A61K45/00(2006.01)i, A61P1/02(2006.01)i, A61P1/16(2006.01)i, A61P11/00(2006.01)i, A61P15/00(2006.01)i, A61P17/00(2006.01)i, A61P35/00(2006.01)i, A61P35/02(2006.01)i, A61P43/00(2006.01)i

Form PCT/ISA/210 (extra sheet) (January 2015)

**INTERNATIONAL SEARCH REPORT**

International application No.
PCT/JP2019/036363

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☐ As all searchable claims could be searched without effort justifying additional fees, this Authority did not invite payment of additional fees.
3. ☒ As only some of the required additional search fees were timely paid by the applicant, this international search report covers only those claims for which fees were paid, specifically claims Nos.: Parts of 1-24

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**

International application No.
PCT/JP2019/036363

<Continuation of Box No. III>

Claim 1 shares the technical feature of an antitumor agent comprising a combination of a quinolinecarboxamide derivative represented by formula (I) or a pharmacologically acceptable salt thereof, and one or more cancer molecular targeted drugs.

However, said technical feature does not make a contribution over the prior art in light of the disclosure of document 1, and thus cannot be said to be a special technical feature. In addition, there do not exist other identical or corresponding special technical features between these inventions.

Thus, the eight inventions below included in claim 1 do not satisfy the unity of invention.

In addition, the structure of "STX-0119" in document 1 is known from documents 2 and 4.

Accordingly, these eight inventions are classified as inventions 1-8.

- Invention 1: Antitumor agent comprising a combination of a quinolinecarboxamide derivative represented by formula (I) or a pharmacologically acceptable salt thereof, and an ALK inhibitor.
- Invention 2: Antitumor agent comprising a combination of a quinolinecarboxamide derivative represented by formula (I) or a pharmacologically acceptable salt thereof, and an EGFR inhibitor.
- Invention 3: Antitumor agent comprising a combination of a quinolinecarboxamide derivative represented by formula (I) or a pharmacologically acceptable salt thereof, and a multi kinase inhibitor.
- Invention 4: Antitumor agent comprising a combination of a quinolinecarboxamide derivative represented by formula (I) or a pharmacologically acceptable salt thereof, and an HER2/EGFR inhibitor.
- Invention 5: Antitumor agent comprising a combination of a quinolinecarboxamide derivative represented by formula (I) or a pharmacologically acceptable salt thereof, and a mTOR inhibitor.
- Invention 6: Antitumor agent comprising a combination of a quinolinecarboxamide derivative represented by formula (I) or a pharmacologically acceptable salt thereof, and a BRAF inhibitor.
- Invention 7: Antitumor agent comprising a combination of a quinolinecarboxamide derivative represented by formula (I) or a pharmacologically acceptable salt thereof, and an MEK inhibitor.
- Invention 8: Antitumor agent comprising a combination of a quinolinecarboxamide derivative represented by formula (I) or a pharmacologically acceptable salt thereof, and a BCR-ABL inhibitor.

In addition, claims 2-13 referring to claim 1, and claims 14-24 having similar statements are classified as corresponding inventions 1-8 by a combination of a quinolinecarboxamide derivative represented by formula (I) and a cancer molecular targeted drug.

The subject of this search was claims 1-3.

Form PCT/ISA/210 (extra sheet) (January 2015)

**INTERNATIONAL SEARCH REPORT**

International application No.
PCT/JP2019/036363

Document 1:
MIYATA, Haruo et al., "Combination of a STAT3 inhibitor and an mTOR inhibitor against a temozolomide-resistant glioblastoma cell line", 2017, vol. 14 (1)
Document 2:
MATSUNO, Kenji et al. "Identification of a New Series of STAT3 Inhibitors by Virtual Screening", ACS Med. Chem. Lett., 2010, vol. 1 (8), pp. 371-375
Document 4:
ISLAM, Rafiqul et al., "Facile Synthesis of 2-Phenylquinoline-4-Carboxamide Derivatives with Variant Structural Features", Heterocycles, 2014, vol. 89 (3), pp. 693-708, fig. 1, scheme 3 (compound 15a-c)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5650529 B


### Non-patent literature cited in the description


- *Pharmacol Rev,* 2006, vol. 58, 621-681